# EUROPEAN PATENT SPECIFICATION

(11) **EP 4 565 302 B1**
(45) Date of publication and mention of the grant of the patent: **07.10.2026**
(21) Application number: 23751918.6
(22) Date of filing: 02.08.2023
(51) Int. Cl.: A61M 15/00

(54) **DRY POWDER INHALER**
TROCKENPULVERINHALATOR
INHALATEUR DE POUDRE SÈCHE

(30) Priority: 03.08.2022 EP 22188457
(43) Date of publication of application: 11.06.2025
(73) Proprietor: Chiesi Farmaceutici S.p.A., 43122 Parma (IT)
(72) Inventor: DI CASTRI, Marco, 43122 PARMA (IT); ABERCROMBIE, Stuart Robert, Cambridge CB4 0DW (GB); TAYLOR, Donal Joseph, Cambridge CB4 0DW (GB)
(74) Representative: PGA S.p.A.
(86) International application number: PCT/EP2023/071410
(87) International publication number: WO 2024/028388

(56) References cited:
- WO-A1-2021/105440
- WO-A2-2008/003701
- US-A1- 2005 183 723
- US-A1- 2014 158 126
- FACCHINETTI FABRIZIO ET AL: "Tanimilast, A Novel Inhaled Pde4 Inhibitor for the Treatment of Asthma and Chronic Obstructive Pulmonary Disease", FRONTIERS IN PHARMACOLOGY, vol. 12, 23 November 2021 (2021-11-23), XP093029049, DOI: 10.3389/fphar.2021.740803

## Description

### Technical field of the invention

The present invention relates to a dry powder inhaler, i.e. a device for dispensing a powdered medicament preparation by inhalation. The device is in particular a portable, multiple-dose, breath activated dry powder inhaler without propellant gas, equipped with a metering device which dispenses doses delivered from a medicament container.

### Background art

Inhalers are hand-held portable devices that deliver medication directly to the lungs. One class of inhalers is passive dry powder inhalers ("DPI"). A passive DPI is a patient driven device wherein the action of breathing in through the device draws the powder formulation of a medicament into the respiratory tract. DPIs are well recognized as devices for drug delivery to the lungs for treatment of pulmonary and systemic diseases. They can generally be divided in: i) single-dose (unit-dose) inhalers, for the administration of an individual dose of the active ingredient/s contained in capsule or blister loaded into the device and punctured by the patient immediately before use; ii) pre-metered multi-dose inhalers containing a series of blisters or capsules with the active ingredient/s formulation or iii) reservoir inhalers containing a larger amount of the powder formulation of active ingredient/s, corresponding to multiple doses, which is metered from a storage unit just before inhalation.

Document WO 2004/012801, by the same Applicant, discloses a reservoir inhaler (type iii) which is a dry powder inhaler comprising a casing with a lower shell and an integral cover pivotably coupled to the lower shell. The lower shell delimits a mouthpiece and the integral cover is movable between a closed position in which the mouthpiece is enclosed and hidden by the integral cover and an open position in which the mouthpiece is exposed for use. The lower shell houses a container for storing a powdered medicament, a metering member having a dosing recess to be filled with a dose of the powdered medicament and an inhalation channel in communication with the mouthpiece. The dosing recess is cup shaped and presents a circular edge. The metering member is moveable between a filling position, in which the dosing recess is in alignment with an opening of the container so as to be filled by gravity with a dose of the powdered medicament, and an inhalation position, in which the dosing recess is in alignment with a vortex chamber connected to an inhalation channel for enabling inhalation of the dose of the powdered medicament contained in the dosing recess. The powder inhaler further comprises a protective member which is slidingly moveable on the metering member between a closed position, in which the protective member covers the dosing recess of the metering member if the metering member is in an inhalation position, and an open position, in which the protective member exposes the dosing recess thereby enabling inhalation of the dose of the powdered medicament contained in the dosing recess. The protective member is coupled to an inhalation actuated mechanism in such a manner that the inhalation actuated mechanism moves the protective member from its closed position to its open position if there is an inhalation suction force exerted by a user which exceeds a predetermined level.

Documents US 2014/158126 A1, WO 2016/000983 and WO 2021/105440, by the same Applicant, discloses a dry powder inhaler (reservoir inhaler) similar to the one of WO 2004/012801. The paper *"*Expert Opin. Drug Deliv. (2014) 11(9), 1497-1506 by Corradi M. *et al."* also discloses a dry powder inhaler (NEXThaler^{®} DPI) by the Applicant.

The dosing recess of the cited prior art documents has a spherical cup shape with a circular edge (in top view) and is usually configured to house a dose of 5-10 mg of powdered medicament. This means that the DPI is configured to deliver such dose through each inhalation.

There could be however the need of delivering powdered medicaments in doses greater than the doses delivered by the prior art inhalers, for instance greater than 10 mg, for each inhalation.

Applicant realized that for doses higher than 10 mg the system of prior art may suffer of some drawbacks and that increasing the diameter of the edge or the height of a spherical cup shaped dosing recess may imply modification of other components of the known DPIs, which should be designed anew and produced through specifically made moulds.

### Summary

The invention is defined in the appended claims. It is an object of the present disclosure to provide a dry powder inhaler of type iii (reservoir inhaler) capable of delivering a dose higher than the dose delivered by the prior art, for instance higher than 10 mg, preferably without changing and/or re-designing most of the components of the known DPIs.

It is also an object of the present disclosure to provide a dry powder inhaler which is able to deliver larger doses maintaining or enhancing the delivery efficiency of the known DPIs.

In particular, it is object of the present disclosure to provide a dry powder inhaler which allows to completely fill the larger dosing recess with the powdered medicament dispensed by gravity from the container.

It is also object of the present disclosure to provide a dry powder inhaler able to completely empty the dosing recess and to ensure the evacuation and delivery of the entire dose through the mouthpiece.

At least one of the above objects is substantially achieved by a dry powder inhaler according to one or more of the appended claims and/or of the following aspects. In accordance with a 1^{st} independent aspect, a dry powder inhaler comprises:
- a casing having a mouthpiece;
- an inhalation channel housed in the casing and connected to the mouthpiece;
- a container housed in the casing for storing a powdered medicament, the container having an opening;
- a de-agglomerator having a vortex chamber located at an end of the inhalation channel opposite the mouthpiece;
- a metering device comprising a shuttle having a dosing recess fashioned in a face of the shuttle, wherein the shuttle is movable between a filling position, in which the dosing recess is in alignment with the opening of the container and faces said opening so as to be filled with a dose of the powdered medicament, and an inhalation position, in which the dosing recess is in alignment with the vortex chamber and the inhalation channel, for enabling inhalation of the dose of the powdered medicament contained in the dosing recess through the mouthpiece;

wherein, in a top view, the opening of the container is elongated along a respective major axis and the dosing recess is elongated along a respective main axis;
wherein, when the shuttle is in the filling position, an edge of the opening of the container encloses the dosing recess.

The dry powder inhaler (DPI) of the disclosure is a reservoir inhaler (type iii) containing a large amount of the powder formulation of active ingredient/s, corresponding to multiple doses, which is metered from a storage unit (container) just before inhalation.

The shape of the dosing recess according to the invention allows to receive from the container and to contain a dose of powdered medicament greater than the dose contained in the spherical cup shaped dosing recess of the prior art.

The shape of the dosing recess according to the disclosure allows to fill completely said dosing recess, because no part of the dosing recess lies outside the edge of the opening of the container.

The invention allows to change only the shape of the dosing recess, i.e. the mould for the shuttle and to keep unchanged the other components of the known DPIs. Therefore, the invention allows to manufacture DPIs able to deliver higher amounts of powdered medicament by employing most of the same elements of the DPIs of the prior art and only a limited number of different elements and by fully exploiting existing production lines.

Other aspects of the disclosure are disclosed in the following. In a 2^{nd} aspect according to aspect 1, a direction of movement of the shuttle between the filling position and the inhalation position and said major axis delimit between them an angle of 90° and said main axis and the major axis delimit between them a first angle other than 0°.

In a 3^{rd} aspect according to aspect 2, said first angle is between 10° and 30°, optionally said first angle is of 20°.

In a 4^{th} aspect according to any of the previous aspects 1 to 3, the de-agglomerator has two air inlets opening in the vortex chamber, said two air inlets being placed on opposite sides of the vortex chamber and along tangential or substantially tangential inflow directions to form an air vortex in said vortex chamber; in the inhalation position, the dosing recess faces the vortex chamber and is fully enclosed in the vortex chamber; optionally, the dosing recess has opposite ends located along the main axis and, in the inhalation position, each of the opposite ends of the dosing recess is next to one of the air inlets.

In a 5^{th} aspect according to aspect 4, a diametrical line connecting the two air inlets and the main axis delimit between them a second angle other than 0°.

In a 6^{th} aspect according to aspect 4 or 5, the de-agglomerator comprises two curved walls having concavities facing each other, the two curved walls being staggered from each other and delimiting the vortex chamber and the two tangential air inlets; optionally, a diametrical line passing through a free extremity of each of the curved walls and the main axis delimit between them the second angle other than 0°.

In a 7^{th} aspect according to aspect 5 or 6, the diametrical line is parallel to the major axis and the first angle is equal to the second angle.

In an 8^{th} aspect according to the any of preceding aspects 4 to 7, each of opposed ends of the dosing recess is located downstream of the respective air inlet with respect to an air inflow entering through said tangential air inlet.

In a 9^{th} aspect according to any of aspects 5 to 7 or to aspect 8 when according to any of aspects 5 to 7, the vortex chamber is configured to form a clockwise air vortex and the main axis is rotated clockwise with respect to the diametrical line or the vortex chamber is configured to form a counterclockwise air vortex and the main axis is rotated counterclockwise with respect to the diametrical line.

In a 10^{th} aspect according to aspect 6 or to any of aspects 7 to 9 when according to aspect 6, in the inhalation position, the dosing recess is contained within a base circle having a diameter "d" given by a segment extending between the free extremities of the two curved walls.

In a 11^{th} aspect according to any of aspects 1 to 10, the edge of the opening of the container is substantially elliptical or the edge of the opening of the container has two major arched sides and two minor straight sides.

In a 12^{th} aspect according to any of aspects 1 to 11, a perimeter of the dosing recess comprises two parallel straight lines connected by two arcs and the two parallel straight lines are parallel to the main axis or the perimeter of the dosing recess is oval or is an ellipse and the main axis is a major axis of the ellipse.

In a 13^{th} aspect according to any of aspects 1 to 12, the dosing recess has a capacity for a powdered medicament of greater than 10 mg, optionally greater than 15 mg, optionally of 20 mg, optionally comprised between 20 mg and 30 mg.

In a 14^{th} aspect according to any of aspects 1 to 13, the dosing recess has a length L measured along the main axis and a width W measured perpendicular to the main axis; optionally, a ratio L/W is greater than 1, optionally between 1.4 and 1.8, e.g. of 1.6.

In a 15^{th} aspect according to any of aspects 1 to 14, a protective member is provided between the shuttle and the vortex chamber; when the shuttle is in the inhalation position, the protective member is slidingly movable on or above the shuttle between a closed position and an open position; in the closed position the protective member fully covers the dosing recess and prevents communication between said dosing recess and the vortex chamber; in the open position the protective member leaves the dosing recess exposed to the vortex chamber.

The shape and position of the dosing recess with respect to the vortex chamber, when the shuttle is in the inhalation position and the protective member is in the open position, ensure a complete emptying of the dosing recess, such that the dose of powdered medicament housed in the dosing recess is entrained in the air flow and directed to the mouthpiece through the inhalation channel.

In a 16^{th} aspect according to aspect 6 or 10, each curved wall is an arc of circumference, optionally a hemi-circumference.

In a 17^{th} aspect according to aspect 10, a width "p" of each air inlet measured along the segment extending between the free extremities of the two curved walls is between d/6 and d/4, optionally the width "p" is d/5.

In a 18^{th} aspect according to aspect 10 or 17, when the shuttle is in the inhalation position, a first minimum distance "s₁" between a perimeter of the dosing recess and the curved walls is between d/12 and d/8, optionally the first minimum distance "s₁" is d/10.

In a 19^{th} aspect according to one or more of the previous aspects 6, 10, 17 or 18, the de-agglomerator comprises a base wall having a through opening for receiving the powder medicament from the dosing recess when the shuttle is in the inhalation position.

In a 20^{th} aspect according to the previous aspect, the two curved walls extend from the base wall and surround or delimit the through opening.

In a 21^{st} aspect according to one or more of the previous aspects 6, 10 or 17 to 20, the end of the inhalation channel opposite the mouthpiece is placed close or between the two curved walls.

In a 22^{nd} aspect according to one or more of the previous aspects 6, 10 or 17 to 21, each of the two curved walls has a radius "R", the two curved walls have respective centers and said centers are staggered of a distance "Δ"; optionally, the respective centers are both on the diametrical line.

In a 23^{rd} aspect according to the previous aspect when according to aspect 17, given a thickness "t" of the curved walls: Δ = t + p and d = 2R - Δ.

In a 24^{th} aspect according to one or more of the previous aspects, the inhalation channel and the vortex chamber share a common central axis.

In a 25^{th} aspect according to aspect 11 or to aspect 12 when according to aspect 11, the two minor straight sides of the edge of the opening are parallel to the direction of movement of the shuttle.

In a 26^{th} aspect according to the aspect 12, the two arcs of the perimeter of the dosing recess define the opposite ends of the dosing recess.

In a 27^{th} aspect according to aspect 12 or 26 when aspect 12 is according to aspect 10, when the shuttle is in the filling position, a second minimum distance "s₂" between the perimeter of the dosing recess and the edge of the opening of the container is between d/12 and d/8, optionally the second minimum distance "s₂" is d/10.

In 28^{th} aspect according to aspect 14, the length L is between 8 and 12 mm.

In 29^{th} aspect according to aspect 14 or 28, the width W is between 4 mm and 6 mm.

In a 30^{th} aspect according to the previous aspects 10 and 14, a ratio L/d is between 0.8 and 0.95, optionally of 0.9.

In a 31^{st} aspect according to the previous aspects 10 and 14 or according to aspect 30, a ratio W/d is between 0.4 and 0.8, optionally of 0.6.

In a 32^{nd} aspect according to any of the previous aspects, the container comprises a medicament chamber storing or configured to store the powdered medicament and having said opening.

In a 33^{rd} aspect according to the previous aspect, the medicament chamber is at least in part shaped like a hopper converging towards the opening.

In a 34^{th} aspect according to aspect 32 or 33, the container comprises a desiccant chamber storing or configured to store a desiccant.

In a 35^{th} aspect according to the previous aspect, a permeable membrane separates the desiccant chamber from the medicament chamber.

In a 36^{th} aspect according to aspect 15, the protective member is a plate.

In a 37^{th} aspect according to aspect 15 or 36, a width of the protective member measured along the major axis is greater than a size of the dosing recess measured along said major axis.

In a 38^{th} aspect according to aspect 15 or 36 or 37, an inhalation actuated mechanism is coupled to the protective member and is configured to move the protective member from its closed position to its open position if there is an inhalation suction force exerted by a user through the mouthpiece which exceeds a predetermined level.

In a 39^{th} aspect according to the previous aspect, the inhalation actuated mechanism comprises an inhalation actuated member, optionally shaped like a flap, a coupling member and a resilient element arranged on the coupling member; the inhalation actuated member is coupled to the protective member through the coupling member such that, if there is an inhalation suction force exceeding a predetermined value, the inhalation actuated member is moved from a first position to a second position, thereby causing the protective member to move from the closed position to the open position.

In a 40^{th} aspect according to any of the previous aspects, the dry powder inhaler further comprises the powdered medicament.

In a 41^{st} aspect according to any of the previous aspects, the dry powder inhaler further comprises a cover engageable with the casing to close the mouthpiece.

In a 42^{nd} aspect according to the previous aspect, the shuttle is mechanically coupled to the cover such that an opening of the cover beyond a range of rotational movement from the closed position causes the shuttle to move from the filling position to the inhalation position and closing of the cover causes the shuttle to move back from the inhalation position to the filling position.

In a 43^{rd} aspect according to any of the previous aspects, the dry powder inhaler is the same as or similar to the devices disclosed in WO 2004/012801 or in WO 2016/000983 or in WO 2021/105440 or in paper *"*Expert Opin. Drug Deliv. (2014) 11(9), 1497-1506 by Corradi M. *et al.",* apart from the shape and size of the dosing recess.

In a 43^{rd} aspect according to any of the previous aspects, the powdered medicament is a pharmaceutical composition.

In a 44^{th} aspect according to the previous aspect, the pharmaceutical composition comprises one or more phosphodiesterase-4 (PDE-4) inhibitors.

In a 45^{th} aspect according to the previous aspect, the phosphodiesterase-4 (PDE-4) inhibitors are selected from the group consisting of tanimilast, cilomilast, roflumilast, tetomilast, oglemilast, apremilast, piclamilast and a salt thereof.

In a 46^{th} aspect according to aspect 43 or 44, the pharmaceutical composition comprises tanimilast or the administered phosphodiesterase-4 (PDE-4) inhibitor is tanimilast.

In accordance with a 47^{th} aspect, a method of treatment of a respiratory disease comprises the administration of a delivered dose through an inhaler according to any of the previous aspects.

In a 48^{th} aspect according to the previous aspect, the delivered dose is greater than 10 mg.

In a 49^{th} aspect according to aspect 47 or 48, the delivered dose is a pharmaceutical composition.

In a 50^{th} aspect according to the previous aspect, the pharmaceutical composition comprises one or more phosphodiesterase-4 (PDE-4) inhibitors.

In a 51^{st} aspect according to the previous aspect, the phosphodiesterase-4 (PDE-4) inhibitors are selected from tanimilast, cilomilast, roflumilast, tetomilast, oglemilast, apremilast, piclamilast and a salt thereof.

In a 52^{nd} aspect according to aspect 49 or 50, the pharmaceutical composition comprises tanimilast or the administered phosphodiesterase-4 (PDE-4) inhibitor is tanimilast.

A 53^{rd} aspect relates to use of the inhaler of any of the preceding aspects 1 to 46 in the treatment of a respiratory disease.

In a 54^{th} aspect according to aspect 52 or 53, the powdered medicament comprises tanimilast.

In a 55^{th} aspect according to any of the previous aspects, the dosing recess has a volume of greater than 10 mm³, optionally greater than 20 mm³, optionally greater than 25 mm³. The dosing recess may have a volume of 10 mm³ to 50 mm³, optionally 20 mm³ to 40 mm³, or optionally 25 mm³ to 35 mm³.

Further features and advantages will be clearer from the detailed description of preferred but not exclusive embodiments of a dry powder inhaler according to the present invention.

### Description of the drawings

Figure 1 shows a 3D view of a dry powder inhaler according to the present invention in an open configuration;
Figures 2A, 2B and 2C are section views of the dry powder inhaler of Figure 1 in different states;
Figures 3 is an exploded view of some components of the dry powder inhaler of Figure 1;
Figure 4 shows a 3D view of one of the components of Figure 3;
Figures 5A and 5B are top views of the component of Figure 4 in different states;
Figure 6 shows a 3D top view of another component of Figure 3;
Figure 7 shows a 3D bottom view of the component of Figure 6;
Figure 8 shows a section view of the component of Figure 6;
Figure 9 is an enlarged top view of a portion of Figure 5B;
Figure 10 is an enlarged top view of another portion of Figure 5A.

### Detailed description

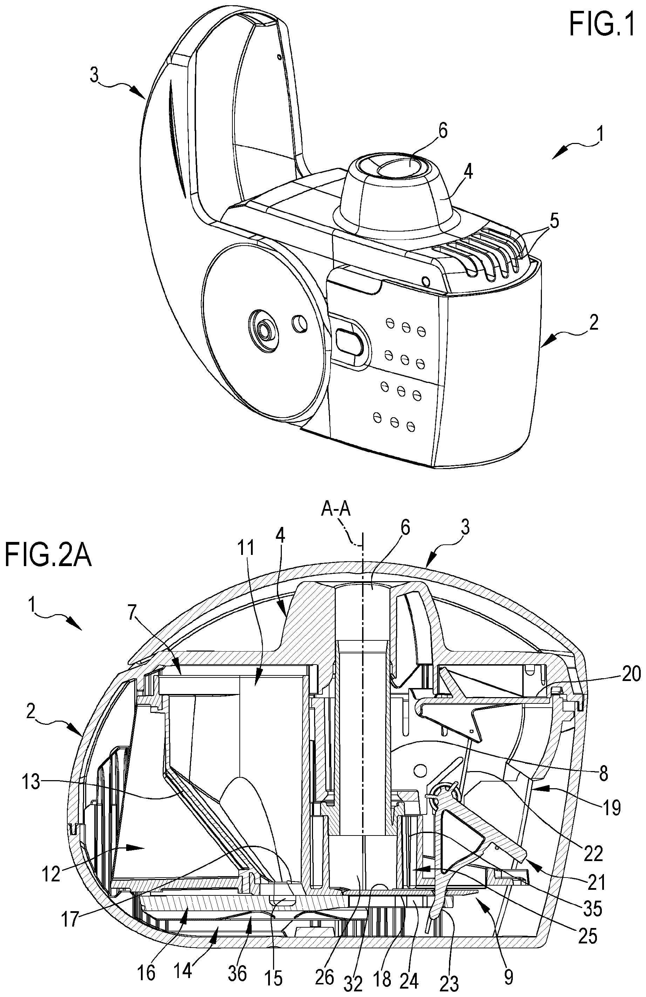

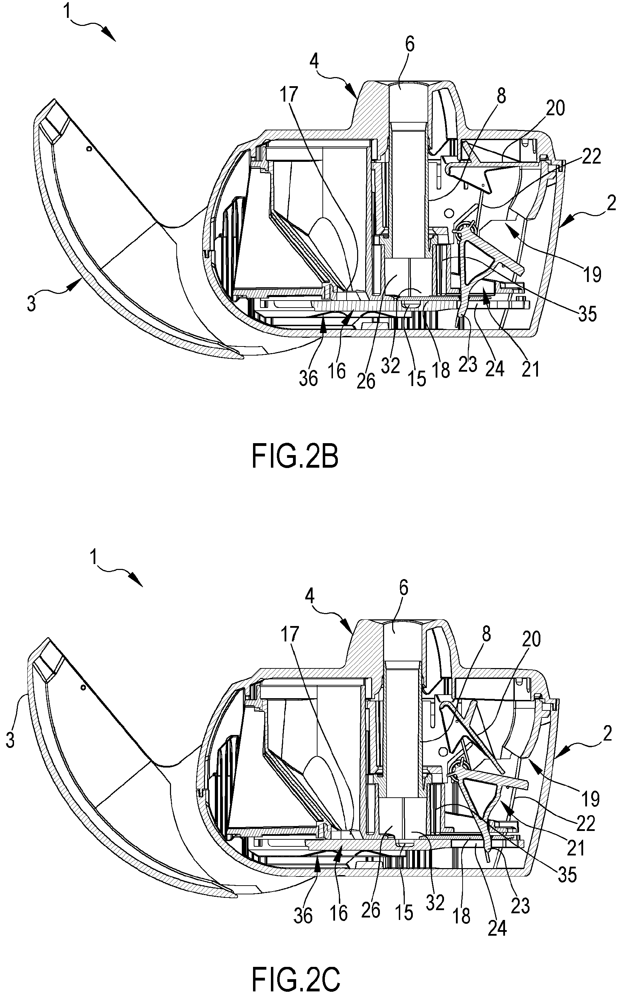

With reference to the appended drawings, Figures 1, 2A, 2B, 2C show an embodiment of a dry powder inhaler 1 according to the present invention. The dry powder inhaler 1 of these non-limiting examples may be similar to the inhalers disclosed in document WO 2004/012801, WO 2016/000983 and WO 2021/105440 or in paper *"*Expert Opin. Drug Deliv. (2014) 11(9), 1497-1506 by Corradi M. *et al."* by the same Applicant.

The dry powder inhaler 1 comprises a casing 2 and a cover 3 being pivotably or rotatably coupled to the casing 2. As can be taken from Figure 1, the cover 3 can be opened to reveal a mouthpiece 4 through which a user can inhale a powdered medicament. At an upper front side of the mouthpiece 4, air intake openings 5 are formed in the casing 2.

The casing 2 is a closed shell made of thermoplastic materials (e.g. ABS and polycarbonate) and comprises lateral sides, an upper side and a lower side (upper and lower with respect to the orientation of the powder inhaler 1 of figures 1, 2A - 2C).

The mouthpiece 4 protrudes from the upper side and has an external shape of truncated cone tapering towards an opening 6 fashioned in a top portion (smaller base) of the mouthpiece 4.

The cover 3 is hinged to the casing 2 and can be rotated between a closed position, shown in Figure 2A, in which the cover 3 encloses the mouthpiece 4, and an open position, shown in Figures 1, 2B and 2C, in which the cover 3 is spaced from the mouthpiece 4 to expose said mouthpiece 4 for use.

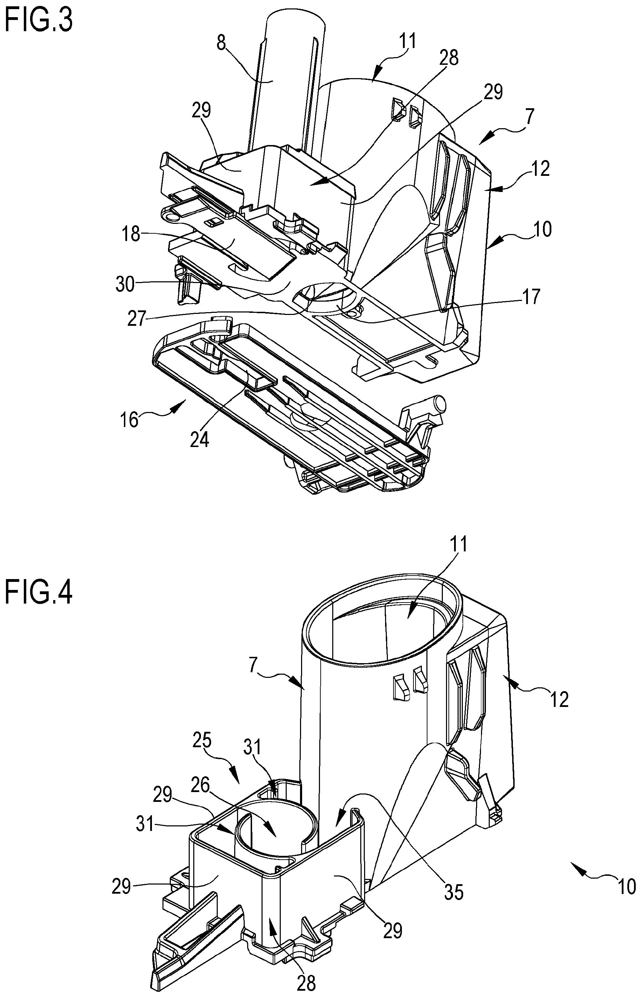

The powder inhaler 1 comprises a container 7 for storing a powdered medicament, an inhalation channel 8 connected to the opening 6 of the mouthpiece 4 and a dispensing device 9 (Figure 2). The inhalation channel 8 has a first opening connected to the mouthpiece 4 and a second opening, opposite with respect to the first opening. All these elements are part of sub-assembly 10, shown in Figure 3, housed inside the casing 2.

As is shown in Figures 2A-2C, the container 7 is a container with integral desiccant. The container 7 comprises a medicament chamber 11 storing the powdered medicament and a desiccant chamber 12 storing a desiccant for absorbing moisture that may have entered the medicament chamber 11.

The desiccant chamber 12 is separated from the medicament chamber 11 by a separate permeable membrane 13. This permeable membrane 13 is of a different permeability than the permeability between either the desiccant or the medicament to the outside environment. The permeability of the membrane 13 can be achieved, for example, by making it of a different material and/or a thinner section than the main body of the container 7. Foils may be used to seal both the medicament chamber 11 and the desiccant chamber 12. The container 7, in particular the medicament chamber 11, is filled or is configured to be filled with an amount of powdered medicament corresponding to a plurality of doses, e.g. up to 100 - 200 doses. For instance, the powdered medicament is a pharmaceutical composition. The desiccant is contained in a housing able of being inserted in the desiccant chamber 12 or the desiccant is in the form of a single tablet able of being inserted in the desiccant chamber 12. The desiccant is or comprises molecular sieves made of a material with pores of uniform size, for instance alkaline salts of aluminosilicates, called zeolites, or aluminophosphates or porous glass or active carbon or artificial zeolites. The molecular sieves are configured to absorb small molecules such as molecules of water. The desiccant may also be a silica gel. The dispensing device 9 comprises a metering device 14 having a dosing recess 15. The metering device 14 shown in the attached Figures comprises a shuttle 16 shaped like a plate and provided with said dosing recess 15 which is fashioned in a face of the shuttle 16.

The dispensing device 9 is movable, with respect to the container 7 and with respect to the inhalation channel 8, between an idle state (Figures 2A), in which a dosing recess 15 is in communication with an opening 17 of the container 7 so as to be filled with a dose of the powdered medicament, and a triggered state (Figure 2C), in which the dosing recess 15 is in communication with the inhalation channel 8 for enabling inhalation of the dose of the powdered medicament contained in the dosing recess 15 through the mouthpiece 4.

The medicament chamber 11 is at least in part shaped like a hopper having walls converging towards the opening 17, as visible in Figures 2A, 2B, 2C, 3 and 5.

The shuttle 16 is placed between the sub-assembly 10 and a bottom wall of the casing 2. The shuttle 16 is shaped like a plate made of a single piece of plastic, e.g. acrylonitrile butadiene styrene copolymer (ABS).

The shuttle 16 is slidingly moveable between a filling position (Figures 2A) and an inhalation position (Figures 2B and 2C) along a direction of movement F. The filling position corresponds to the idle state of the metering device 14, in which the dosing recess 15 is in alignment with the opening 17 of the container 7 and faces said opening 17 so as to be filled with the dose of the powdered medicament. The inhalation position corresponds to an armed state (Figure 2B) which will be detailed later and to the triggered state (Figure 2C) of the metering device 14, in which the dosing recess 15 is in alignment with the inhalation channel 8.

The shuttle 16 is mechanically coupled to the cover 3 such that an opening of the cover 3 beyond a range of rotational movement from the closed position causes the shuttle 16 to move from the filling position to the inhalation position. Closing of the cover 3 causes the shuttle 16 to move back from the inhalation position to the filling position.

As shown in Figures 2A, 2B and 2C, a spring 36 is interposed between a bottom wall of the casing 2 and the shuttle 16 and is configured to push said shuttle 16 against the opening 17 of the container 7 when the shuttle 16 is in the filling position. The shuttle 16 slides with respect to the spring 36 when moving between the filling position and the inhalation position.

The metering device 14 further comprises a protective member 18 provided between the shuttle 16 and the inhalation channel 8. The protective member 18 is a plate arranged between the second opening of the inhalation channel 8 and the shuttle 16. The protective member 18 is parallel with respect to the shuttle 16 and is slidingly movable on or above the shuttle 16 between a closed position and an open position.

In the closed position, the protective member 18 is shifted backwards towards the second opening of the inhalation channel 8 and towards the container 7. In the closed position, a rear part of the protective member 18 may at least in part close the second opening of the inhalation channel 8. In the open position, the protective member 18 is shifted forward towards a wall of the casing 2. In the open position, a rear part of the protective member 18 leaves the second opening of the inhalation channel 8 open. The protective member 18 is in the closed position when the shuttle 16 is in the filling position (Figures 2A). The protective member 18 may be moved between the closed position and the open position when the shuttle 16 is in the inhalation position (Figures 2B and 2C).

Therefore, the metering device 14 is configured to take the three different states cited above (idle, armed, triggered) and these states are determined by the positions of the shuttle 16 and of the protective member 18.

In the idle state (Figure 2A), the shuttle 16 is in the filling position and the protective member 18 is in the closed position. The protective member 18 does not cover the dosing recess 15. The dosing recess 15 is communication with the opening of the container 7 to receive the medicament dose. In the armed state (Figure 2B), the shuttle 16 is in the inhalation position and the protective member 18 is in the closed position. The protective member 18 covers the dosing recess 15. The protective member 18 prevents the powdered medicament contained in the dosing recess 15 from entering the inhalation channel 8 and being lost in case of rotation or movement of the powder inhaler 1 in oblique position before the inhalation manoeuvre or if the user blows into the mouthpiece 4. In the triggered state (Figure 2C), the shuttle 16 is in the inhalation position and the protective member 18 is in the open position. The protective member 18 does not cover the dosing recess 15, thereby exposing the dosing recess 15 to the inhalation channel 8 so as to enable a user to inhale the dose of the powdered medicament contained in the dosing recess 15.

The dispensing device 9 further comprises a breath or inhalation actuated mechanism 19 coupled to the protective member 18 (Figures 2A, 2B, 2C). The inhalation actuated mechanism 19 comprises an inhalation actuated member 20 shaped like a flap, a coupling member 21 and a resilient element 22 (spring) arranged on the coupling member 21.

The flap 20 is coupled to the protective member 18 through the coupling member 21 such that, if there is an inhalation suction force exceeding a predetermined value, the flap 20 is moved from a first position to a second position, thereby causing the protective member 18 to move from the closed position to the open position. The flap 20 is placed inside the casing 2 and close to the air intake openings 5. In the first position (Figure 2A), the flap 20 separates the air intake openings 5 from the inhalation channel 8 and seats in a main airflow path. The flap 20 provides a resistance if the user blows into the device giving positive feedback. In the second position, (Figure 2C) the flap 20 is rotated with respect to the first position to open the air intake openings 5 and to allow air flowing through the air intake openings 5 into the inhalation channel 8 and out of the mouthpiece 4. The resilient element 22 is arranged such that said resilient element 22 holds the flap 20 in its first position. When the shuttle 16 is pushed forward by opening the cover 3, the resilient element 22 is compressed and charged and the reset force exerted on the flap 20 is released, so that the flap 20 can pivot or rotate from the first position into the second position that is pivoted downward relative to the first position if there is a sufficient high inhalation suction force in the inhalation channel 8.

The flap 20 is hinged to the casing 2 in order to rotate between the first position and the second position around a respective rotation axis which is substantially perpendicular to a main axis A-A of the inhalation channel 8. The coupling member 21 is also hinged to the casing 2 in order to rotate between a respective first position and second position around a respective rotation axis which is substantially perpendicular to the main axis A-A of the inhalation channel 8.

The coupling member 21 comprises an arm, not shown, protruding towards the flap 20 and engaged with the flap 20 such that the clockwise rotation of the flap 20 from the first position to the second position causes a counterclockwise rotation of the coupling member 21 from its respective first position towards its respective second position.

The coupling member 21 comprises a prolongation 23 engaging with an opening formed in the protective member 18 in order to move the protective member 18 from the closed position to the open position when the coupling member 21 moves from its respective first position to its respective second position and vice-versa.

The prolongation 23 of the coupling member 21 is also moveably arranged in a longitudinal opening 24 which is formed in the shuttle 16 along its longitudinal direction, such that said prolongation 23 can freely move in the longitudinal opening 24, while a movement of the shuttle 16 from the inhalation position to the filling position causes the prolongation 23 of the coupling member 21 to abut against an edge of the longitudinal opening 24 thereby moving the coupling member 21 back into its initial first position.

The dry powder inhaler 1 further comprises a de-agglomerator 25 that is coupled to the second end of the inhalation channel 8 opposite the mouthpiece 4. Also the de-agglomerator 25 is part of the sub-assembly 10.

The de-agglomerator 25 delimits a vortex chamber 26 and is constructed such that it generates a cyclonic airflow resulting in a strong velocity gradient. The protective member 18 is slidable on the shuttle 16 between its closed position, in which is covers the dosing recess 15, and its open position, in which it exposes the dosing recess 15 to the de-agglomerator 25 and the inhalation channel 8 when the metering member 14 is in the inhalation position, so that the dose of the powdered medicament can be inhaled through the de-agglomerator 25 and the inhalation channel 8 as well as the mouthpiece 4.

The powder inhaler 1 may also comprise a dose counting unit, not shown in the embodiment of the attached drawings, contained into the casing 2 and coupled both to the inhalation actuated mechanism 19 and to the closure of the cover 3 after an efficacious inhalation has occurred. The casing 2 may also comprise a window or an opening for displaying the number of doses taken or the number of doses left in the container 7, this number being counted by the dose counting unit.

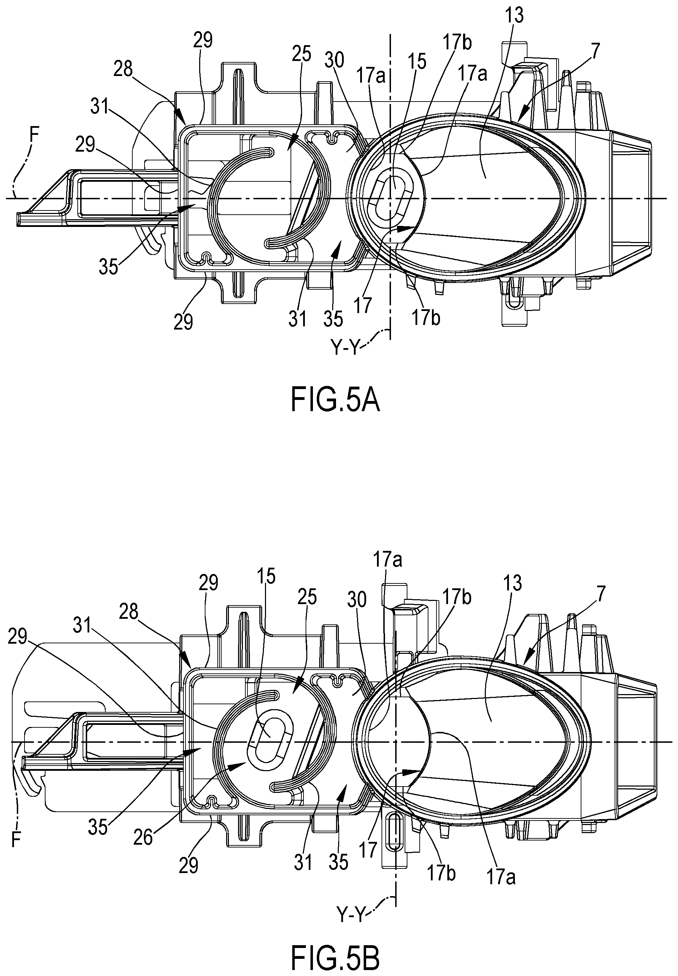

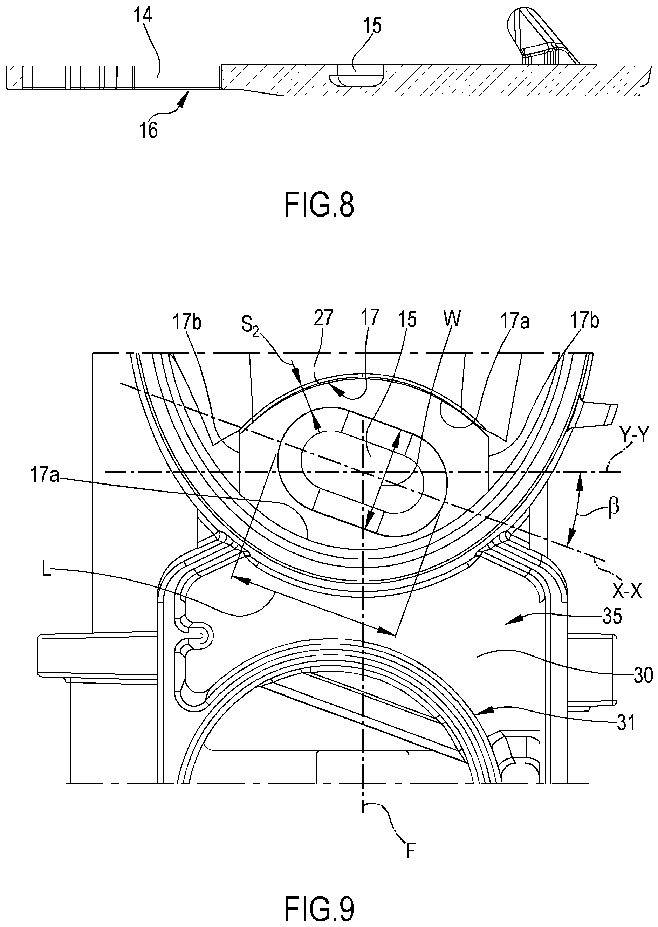

As shown in the top views of Figures 5A, 5B and 9, the opening 17 of the container 7 is elongated along a major axis Y-Y and said major axis Y-Y is perpendicular to the direction of movement F of the shuttle 16. In other words, the opening 17 has a size measured along said major axis greater than a size measured along the direction of movement F of the shuttle 16.

An edge 27 of the opening 17 lies in a plane parallel to the face of the shuttle 16 in which the dosing recess 16 is fashioned (Figures 3, 5A, 5B, 9). The edge 27 is formed by two major arched sides 17a and two minor straight sides 17b. The two minor straight sides 17b present a same length and are parallel to the direction of movement F of the shuttle 16. Each of the two major arched sides 17a connects extremities of the two minor straight sides 17b. The two major arched sides 17a have concavities facing each other. The edge 27 of the opening 17 looks like an oval with truncated ends.

In other embodiments, not shown, the edge 27 of the opening 17 may be elliptical or substantially elliptical.

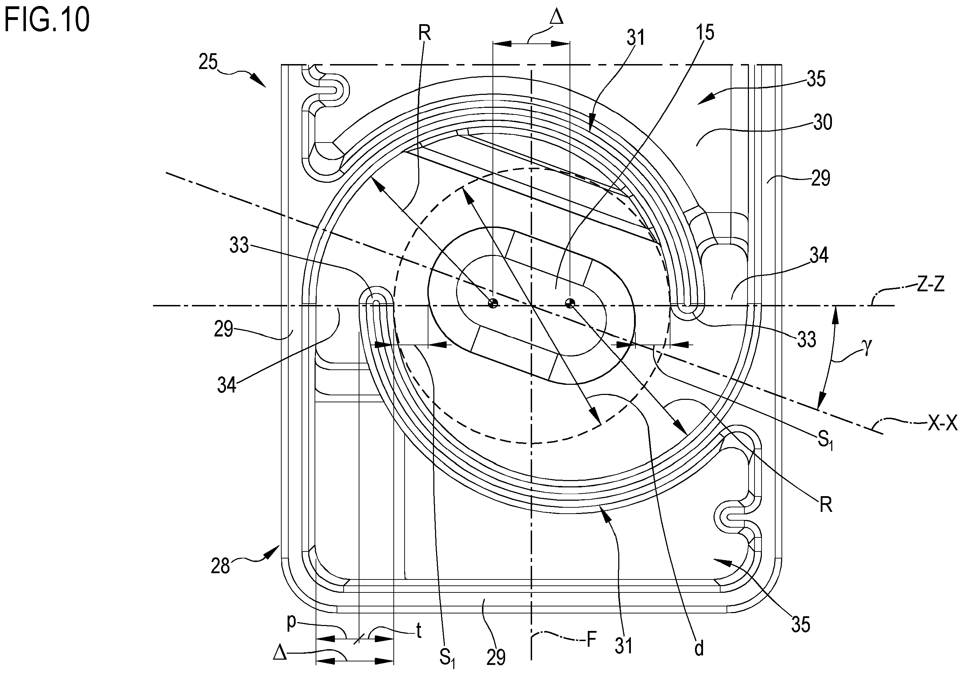

The de-agglomerator 25 comprises a casing 28 (see Figure 3) having lateral walls 29 and a base wall 30 and the vortex chamber 26 is delimited inside said casing 28. As shown in Figures 4, 5A, 5B and 10, the de-agglomerator 25 comprises two curved walls 31 protruding from the base wall 30 of the sub-assembly 10. The two curved walls 31 have concavities facing each other and delimit the vortex chamber 26. Each curved wall 31 has a thickness t and is shaped like a hemi-circumference having a radius R. The centers of the two curved walls 31 are located on a common diametrical line Z-Z and are staggered from each other of a distance Δ (Figure 10). The diametrical line Z-Z is perpendicular to the direction of movement F of the shuttle 16 and therefore parallel to the major axis Y-Y.

The two curved walls 31 extend from the base wall 30 and surround or delimit a through opening 32 fashioned in said base wall 30 (Figures 2A, 2B, 2C).

Each of the two curved walls 31 has an extremity connected to a respective lateral wall 29 and an opposite free extremity 33. A diameter d given by a segment extending between the free extremities 33 of the two curved walls 31 may be considered as the diameter of the vortex chamber 26.

Each free extremity 33 and an adjacent lateral wall 29 delimit an air inlet 34 which opens in the vortex chamber 26. The two air inlets 34 are placed on opposite sides of the vortex chamber and along tangential or substantially tangential inflow directions to form an air vortex in said vortex chamber 26. In other words, the airflow entering through each of the two air inlets 33 is directed tangentially with respect to a circle centered in the vortex chamber 26.

A width p of each air inlet 34 measured along the segment extending between the free extremities 33 of the two curved walls 31 is between d/6 and d/4, e. g. the width p is d/5.

The relations between the radius R, the distance Δ, the width p, the diameter d and the thickness t are the following: $Δ = t + p$ $d = 2 R - Δ$

Hollows 35 delimited between the lateral walls 29 and radially external surfaces of the two curved walls 31 can be put in fluid communication with the air intake openings 5 through the breath or inhalation actuated mechanism 19.

As shown in Figures 2A, 2B and 2C, the second end of the inhalation channel 8, opposite the mouthpiece 4, is placed between the two curved walls 31 and opens into the vortex chamber 26. The inhalation channel 8 and the vortex chamber 26 share a common central axis.

When the user exerts the inhalation suction force exceeding the predetermined value, the protective member 18 moves from the closed position to the open position. In the open position the protective member 18 leaves the dosing recess 15 exposed to the vortex chamber 26. The air flows through the air intake openings 5, into the hollows 35, through the air inlets 34, into the vortex chamber 26 and then into the inhalation channel 8 and out of the mouthpiece 4. The dose of powdered medicament housed in the dosing recess 15 is entrained in the swirling air flow and directed to the mouthpiece 4 through the inhalation channel 8.

As it is evident from the above, the internal mechanisms and functioning of the powder inhaler 1 disclosed above may be substantially the same as those disclosed in documents WO 2004/012801, WO 2016/000983 or WO 2021/105440 by the same Applicant.

The main difference with respect to these documents is the shape, size and positioning of the dosing recess 15, as will be detailed below.

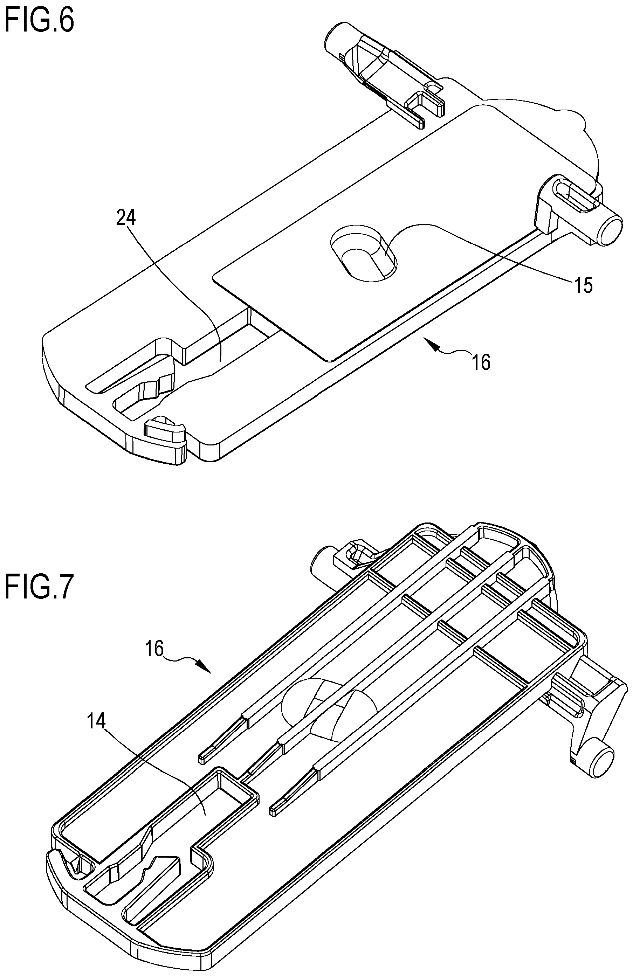

According to the present invention, as shown in the top views of Figures 5A, 5B, 6 and 9, the dosing recess 15 does not present a circular perimeter. Differently, the dosing recess 15 of the invention is elongated along a respective main axis X-X. The dosing recess 15 may be cup shaped.

The shape of the perimeter of the dosing recess 15 may be oval or elliptical and the main axis X-X is a major axis of the ellipse. Otherwise, as shown in the attached Figures 5-9, the perimeter of the dosing recess 15 comprises two parallel straight lines connected by two arcs and the two parallel straight lines are parallel to the main axis X-X. The two arcs of the perimeter of the dosing recess 15 define opposite ends of the dosing recess 15 located along the main axis X-X. The dosing recess has a length L measured along the main axis X-X and a width W measured perpendicular to the main axis X-X and a ratio L/W is greater than 1, preferably between 1.4 and 1.8, e.g. of 1.6.

Furthermore, the main axis X-X and the major axis Y-Y delimit between them a first angle β other than 0°. Said first angle β may be of about 20°.

The size of the perimeter of the dosing recess 15 is such that, when the shuttle 16 is in the filling position, the edge 27 of the opening 17 of the container 7 encloses the dosing recess 15 or, in other words, the perimeter of the dosing recess 15 is surrounded by or enclosed within the edge 27 of the opening 17, as shown in Figure 9. This way, the dosing recess 15 is completely filled by the powdered medicament flowing from the opening 17.

This shape of the dosing recess 15 allows to receive from the opening 17 of the container 7 and to house a dose of powdered medicament greater than the dose contained in the spherical cup shaped dosing recess of the prior art. For instance, the length L is between 8 and 12 mm, the width W is between 4 mm and 6 mm and the capacity of the dosing recess 15 according to the invention is greater than 15 mg and is, for instance, of 20 mg. For instance, the dosing recess 15 may have a volume of greater than 20 mm³, for instance about 30 mm³ or about 32 mm³.

In the inhalation position and when the protective member 18 is in the open position, the dosing recess 15 faces the vortex chamber 26 and is fully in the vortex chamber 26. In particular, the dosing recess 15 is contained within the base circle having the diameter d (Figure 10). The diametrical line Z-Z passing through the free extremity 33 of each of the curved walls 31 and connecting the two air inlets 34 delimits with the main axis X-X a second angle γ which is equal to the first angle β, i.e. other than 0°, for instance of 20°.

Each of the opposite ends of the dosing recess 15 is next/close to one of the air inlets 34 and is located downstream of the respective air inlet 34 with respect to an air inflow entering through said tangential air inlet 34. In the illustrated embodiment, the vortex chamber 26 is configured to form a clockwise air vortex and the main axis X-X is rotated clockwise of the second angle γ with respect to the diametrical line Z-Z. In other embodiments, not shown, the vortex chamber 26 may be configured to form a counterclockwise air vortex and the main axis X-X would be rotated counterclockwise with respect to the diametrical line Z-Z.

When the shuttle 16 is in the inhalation position, a first minimum distance s₁ between the perimeter of the dosing recess 15 and the curved walls 31 is between d/12 and d/8, e.g. the first minimum distance s₁ is d/10. In the attached figure 10, the first minimum distance s₁ is measured along the diametrical line Z-Z.

When the shuttle 16 is in the filling position, a second minimum distance s₂ between the perimeter of the dosing recess 15 and the edge 27 of the opening 17 of the container 7 is between d/12 and d/8, e.g. the second minimum distance s₂ is d/10. A ratio L/d is between 0.8 and 0.95, e.g. of 0.9 and a ratio W/d is between 0.4 and 0.8, e.g. of 0.6. In the attached figure 9, the second minimum distance s₂ is between one of the two major arched sides 17a of the edge 27 and one of the two arcs of the perimeter of the dosing recess 15.

The shape and position of the dosing recess 15 with respect to the vortex chamber 26, when the shuttle 16 is in the inhalation position and the protective member 18 is in the open position, ensure a complete emptying of the dosing recess 15 thanks to the air swirling in the vortex chamber 26. The elongated shape of the dosing recess 15 and its relative position with respect to the air inlets 34 of the vortex chamber 26 allow to collect all the powdered medicament from the dosing recess 15 and to route said powdered medicament into the inhalation channel 8.

Furthermore, a width of the protective member 18 measured along the major axis Y-Y is greater than a size of the dosing recess 15 measured along said major axis Y-Y and a length of the protective member 18 measured along the direction of movement F is greater than a size of the dosing recess 15 measured along said direction of movement F, such that any leak of powdered medicament from the dosing recess 15 and before the inhalation is prevented.

The dry powder inhaler 1 according to the disclosure may be used with all pharmaceutical compositions that can be dispensed through DPIs with a delivered dose greater than 10 mg per actuation.

In particular, the dry powder inhaler 1 according to the disclosure may be used in the treatment of respiratory diseases wherein a delivered dose of a pharmaceutical composition greater than 10 mg per actuation is required.

In a preferred embodiment, the powdered medicament stored in the container 7 of the dry powder inhaler 1 according to the disclosure is a pharmaceutical composition comprising one or more phosphodiesterase-4 (PDE-4) inhibitors selected from the group consisting of tanimilast, cilomilast, roflumilast, tetomilast, oglemilast, apremilast, piclamilast and a salt thereof or other suitable active ingredients. In a more preferred embodiment, the powdered medicament is a pharmaceutical composition comprising tanimilast.

### List of parts

powder inhaler 1
casing 2
cover 3
mouthpiece 4
air intake openings 5
opening 6 of the mouthpiece
container 7
inhalation channel 8
dispensing device 9
sub-assembly 10
medicament chamber 11
desiccant chamber 12
permeable membrane 13
metering device 14
dosing recess 15
shuttle 16
opening 17 of the container
major arched sides 17a
minor straight sides 17b
protective member 18
breath or inhalation actuated mechanism 19
inhalation actuated member or flap 20
coupling member 21
resilient element 22
prolongation 23
longitudinal opening 24
de-agglomerator 25
vortex chamber 26
edge 27 of the opening
casing 28
lateral walls 29
base wall 30
two curved walls 31
through opening 32
free extremity 33 of the lateral wall
air inlets 34
hollows 35
spring 36
direction of movement F of the shuttle
major axis Y-Y
main axis X-X
first angle β
second angle γ
distance Δ
first minimum distance s₁
second minimum distance s₂

## Claims

1. Dry powder inhaler, comprising:
- a casing (2) having a mouthpiece (4);
- an inhalation channel (8) housed in the casing (2) and connected to the mouthpiece (4);
- a container (7) housed in the casing (2) for storing a powdered medicament, the container (7) having an opening (17);
- a de-agglomerator (25) having a vortex chamber (26) located at an end of the inhalation channel (8) opposite the mouthpiece (4);
- a metering device (14) comprising a shuttle (16) having a dosing recess (15) fashioned in a face of the shuttle (16), wherein the shuttle (16) is movable between a filling position, in which the dosing recess (15) is in alignment with the opening (17) of the container (7) and faces said opening (17) so as to be filled with a dose of the powdered medicament, and an inhalation position, in which the dosing recess (15) is in alignment with the vortex chamber (26) and the inhalation channel (8), for enabling inhalation of the dose of the powdered medicament contained in the dosing recess (15) through the mouthpiece (4);
**characterized in that**, in a top view, the opening (17) of the container (7) is elongated along a respective major axis (Y-Y) and the dosing recess (15) is elongated along a respective main axis (X-X); wherein, when the shuttle (16) is in the filling position, an edge (27) of the opening (17) of the container (7) encloses the dosing recess (15).

2. The inhaler of claim 1, wherein a direction of movement (F) of the shuttle (16) between the filling position and the inhalation position and said major axis (Y-Y) delimit between them an angle of 90° and wherein said main axis (X-X) and the major axis (Y-Y) delimit between them a first angle (β) other than 0°.

3. The inhaler of claim 2, wherein said first angle (β) is between 10° and 30°.

4. The inhaler of any of claims 1 to 3, wherein the de-agglomerator (25) has two air inlets (34) opening in the vortex chamber (26), said two air inlets (34) being placed on opposite sides of the vortex chamber (26) and along tangential or substantially tangential inflow directions to form an air vortex in said vortex chamber (26); wherein, in the inhalation position, the dosing recess (15) faces the vortex chamber (26) and is fully enclosed in the vortex chamber (26); wherein the dosing recess (15) has opposite ends located along the main axis (X-X); wherein, in the inhalation position, each of the opposite ends of the dosing recess (15) is next to one of the air inlets (34).

5. The inhaler of claim 4, wherein a diametrical line (Z-Z) connecting the two air inlets (34) and the main axis (X-X) delimit between them a second angle (γ) other than 0°.

6. The inhaler of claim 4, wherein the de-agglomerator (25) comprises two curved walls (31) having concavities facing each other, the two curved walls (31) being staggered from each other and delimiting the vortex chamber (26) and the two tangential air inlets (34); wherein a diametrical line (Z-Z) passing through a free extremity (33) of each of the curved walls (31) and the main axis (X-X) delimit between them a second angle (γ) other than 0°.

7. The inhaler of claim 5 or 6, wherein the diametrical line (Z-Z) is parallel to the major axis (Y-Y) and the first angle (β) is equal to the second angle (γ).

8. The inhaler of any of claims 4 to 7, wherein each of the opposed ends of the dosing recess (15) is located downstream of the respective air inlet (34) with respect to an air inflow entering through said air inlet (34).

9. The inhaler of any of claims 1 to 8, wherein the edge (27) of the opening (17) of the container (7) is substantially elliptical; or wherein the edge (27) of the opening (17) of the container (7) has two major arched sides and two minor straight sides.

10. The inhaler of any of claims 1 to 9, wherein a perimeter of the dosing recess (15) comprises two parallel straight lines connected by two arcs and the two parallel straight lines are parallel to the main axis (X-X); or wherein the perimeter of the dosing recess (15) is oval or is an ellipse and the main axis (X-X) is a major axis of the ellipse.

11. The inhaler of any of claims 1 to 10, wherein the dosing recess (15) has a capacity for the powdered medicament greater than 10 mg, optionally greater than 15 mg, optionally of 20 mg; wherein the dosing recess (15) has a volume of greater than 20 mm³, optionally 20 mm³ to 40 mm³, or optionally 25 mm³ to 35 mm³.

12. The inhaler of any of claims 1 to 11, wherein the dosing recess (15) has a length (L) measured along the main axis (X-X) and a width (W) measured perpendicular to the main axis (X-X); wherein a ratio L/W is greater than 1, optionally between 1.4 and 1.8.

13. The inhaler of any of claims 1 to 12, further comprising a protective member (18) provided between the shuttle (16) and the vortex chamber (26); when the shuttle (16) is in the inhalation position, the protective member (18) is slidingly movable on or above the shuttle (16) between a closed position and an open position; wherein in the closed position the protective member (18) fully covers the dosing recess (15) preventing communication between said dosing recess (15) and the vortex chamber (26); wherein in the open position the protective member (18) leaves the dosing recess (15) exposed to the vortex chamber (26).

14. The inhaler of any of claims 1 to 13, comprising the powdered medicament, wherein said powdered medicament is a pharmaceutical composition comprising one or more phosphodiesterase-4 (PDE-4) inhibitors selected from the group consisting of tanimilast, cilomilast, roflumilast, tetomilast, oglemilast, apremilast, piclamilast and a salt thereof.

15. The inhaler of claim 14, wherein the pharmaceutical composition comprises tanimilast.

## Patentansprüche

1. Trockenpulverinhalator, umfassend:
- ein Gehäuse (2) mit einem Mundstück (4);
- einen Inhalationskanal (8), welcher in dem Gehäuse (2) untergebracht und mit dem Mundstück (4) verbunden ist;
- einen Behälter (7), welcher in dem Gehäuse (2) untergebracht ist, zum Lagern eines pulverförmigen Arzneimittels, wobei der Behälter (7) eine Öffnung (17) aufweist;
- einen Entagglomerator (25) mit einer Wirbelkammer (26), welcher an einem Ende des Inhalationskanals (8) angeordnet ist, welches dem Mundstück (4) entgegengesetzt ist;
- eine Dosiervorrichtung (14) umfassend einen Schieber (16), welcher eine Dosierausnehmung (15) aufweist, welche in einer Fläche des Schiebers (16) ausgebildet ist, wobei der Schieber (16) zwischen einer Füllposition, in welcher die Dosierausnehmung (15) in Flucht mit der Öffnung (17) des Behälters (7) steht und der Öffnung (17) zugewandt ist, um mit einer Dosis des pulverförmigen Arzneimittels gefüllt zu werden, und einer Inhalationsposition, in welcher die Dosierausnehmung (15) in Flucht mit der Wirbelkammer (26) und dem Inhalationskanal (8) steht, um eine Inhalation der in der Dosierausnehmung (15) enthaltenen Dosis des pulverförmigen Arzneimittels durch das Mundstück (4) zu erlauben;
**dadurch gekennzeichnet, dass** in einer Draufsicht die Öffnung (17) des Behälters (7) sich entlang einer jeweiligen großen Achse (Y-Y) ausdehnt und die Dosierausnehmung (15) sich entlang einer jeweiligen Hauptachse (X-X) ausdehnt; wobei, wenn sich der Schieber (16) in der Füllposition befindet, ein Rand (27) der Öffnung (17) des Behälters (7) die Dosierausnehmung (15) umschließt.

2. Inhalator nach Anspruch 1, wobei eine Bewegungsrichtung (F) des Schiebers (16) zwischen der Füllposition und der Inhalationsposition und die große Achse (Y-Y) zwischen sich einen Winkel von 90° begrenzen und wobei die Hauptachse (X-X) und die große Achse (Y-Y) zwischen sich einen ersten Winkel (β) ungleich 0° begrenzen.

3. Inhalator nach Anspruch 2, wobei der erste Winkel (β) zwischen 10° und 30° liegt.

4. Inhalator nach einem der Ansprüche 1 bis 3, wobei der Entagglomerator (25) zwei Lufteinlässe (34) aufweist, welche sich in die Wirbelkammer (26) öffnen, wobei die zwei Lufteinlässe (34) auf entgegengesetzten Seiten der Wirbelkammer (26) und entlang tangentialer oder im Wesentlichen tangentialer Einströmrichtungen platziert sind, um einen Luftwirbel in der Wirbelkammer (26) zu bilden; wobei in der Inhalationsposition die Dosierausnehmung (15) der Wirbelkammer (26) zugewandt ist und vollständig von der Wirbelkammer (26) umschlossen ist; wobei die Dosierausnehmung (15) entgegengesetzte Enden aufweist, welche entlang der Hauptachse (X-X) angeordnet sind; wobei in der Inhalationsposition jedes der entgegengesetzten Enden der Dosierausnehmung (15) neben einem der Lufteinlässe (34) liegt.

5. Inhalator nach Anspruch 4, wobei eine diametrische Linie (Z-Z), welche die zwei Lufteinlässe (34) verbindet, und die Hauptachse (X-X) zwischen sich einen zweiten Winkel (γ) ungleich 0° begrenzen.

6. Inhalator nach Anspruch 4, wobei der Entagglomerator (25) zwei gekrümmte Wände (31) mit einander zugewandten Konkavitäten umfasst, wobei die zwei gekrümmten Wände (31) versetzt zueinander sind und die Wirbelkammer (26) und die zwei tangentialen Lufteinlässe (34) begrenzen; wobei eine diametrische Linie (Z-Z), welche durch eine freie Extremität (33) jeder der gekrümmten Wände (31) verläuft, und die Hauptachse (X-X) zwischen sich einen zweiten Winkel (γ) begrenzen, welcher ungleich 0° ist.

7. Inhalator nach Anspruch 5 oder 6, wobei die diametrische Linie (Z-Z) parallel zu der großen Achse (Y-Y) verläuft und der erste Winkel (β) gleich dem zweiten Winkel (γ) ist.

8. Inhalator nach einem der Ansprüche 4 bis 7, wobei jedes der entgegengesetzten Enden der Dosierausnehmung (15) stromabwärts des jeweiligen Lufteinlasses (34) in Bezug auf einen durch den Lufteinlass (34) eintretenden Lufteinstrom angeordnet ist.

9. Inhalator nach einem der Ansprüche 1 bis 8, wobei der Rand (27) der Öffnung (17) des Behälters (7) im Wesentlichen elliptisch ist; oder wobei der Rand (27) der Öffnung (17) des Behälters (7) zwei große, gewölbte Seiten und zwei kleine, gerade Seiten aufweist.

10. Inhalator nach einem der Ansprüche 1 bis 9, wobei ein Umfang der Dosierausnehmung (15) zwei parallele gerade Linien umfasst, welche durch zwei Bögen verbunden sind, und die zwei parallelen geraden Linien parallel zu der Hauptachse (X-X) sind; oder wobei der Umfang der Dosierausnehmung (15) oval ist oder eine Ellipse ist und die Hauptachse (X-X) eine große Achse der Ellipse ist.

11. Inhalator nach einem der Ansprüche 1 bis 10, wobei die Dosierausnehmung (15) ein Fassungsvermögen für das pulverförmige Arzneimittel von größer als 10 mg, gegebenenfalls größer als 15 mg, gegebenenfalls von 20 mg aufweist; wobei die Dosierausnehmung (15) ein Volumen von größer als 20 mm³, gegebenenfalls 20 mm³ bis 40 mm³ oder gegebenenfalls 25 mm³ bis 35 mm³ aufweist.

12. Inhalator nach einem der Ansprüche 1 bis 11, wobei die Dosierausnehmung (15) eine entlang der Hauptachse (X-X) gemessene Länge (L) und eine senkrecht zu der Hauptachse (X-X) gemessene Breite (W) aufweist; wobei ein Verhältnis L/W größer als 1 ist, gegebenenfalls zwischen 1,4 und 1,8 liegt.

13. Inhalator nach einem der Ansprüche 1 bis 12, ferner umfassend ein Schutzelement (18), welches zwischen dem Schieber (16) und der Wirbelkammer (26) bereitgestellt ist; wobei, wenn sich der Schieber (16) in der Inhalationsposition befindet, das Schutzelement (18) an oder über dem Schieber (16) zwischen einer geschlossenen Position und einer offenen Position gleitend bewegbar ist; wobei das Schutzelement (18) in der geschlossenen Position die Dosierausnehmung (15) vollständig bedeckt, um Kommunikation zwischen der Dosierausnehmung (15) und der Wirbelkammer (26) zu verhindern; wobei das Schutzelement (18) in der offenen Position die Dosierausnehmung (15) gegenüber der Wirbelkammer (26) exponiert lässt.

14. Inhalator nach einem der Ansprüche 1 bis 13, umfassend das pulverförmige Arzneimittel, wobei das pulverförmige Arzneimittel eine pharmazeutische Zusammensetzung ist, welche einen oder mehrere Phosphodiesterase-4 (PDE-4)-Inhibitoren umfasst, welche ausgewählt sind aus der Gruppe bestehend aus Tanimilast, Cilomilast, Roflumilast, Tetomilast, Oglemilast, Apremilast, Piclamilast und einem Salz davon.

15. Inhalator nach Anspruch 14, wobei die pharmazeutische Zusammensetzung Tanimilast umfasst.

## Revendications

1. Inhalateur de poudre sèche, comprenant :
- un boîtier (2) ayant un embout buccal (4) ;
- un canal d'inhalation (8) logé dans le boîtier (2) et raccordé à l'embout buccal (4) ;
- un récipient (7) logé dans le boîtier (2) pour stocker un médicament en poudre, le récipient (7) ayant une ouverture (17) ;
- un désagglomérateur (25) ayant une chambre à vortex (26) localisée à une extrémité du canal d'inhalation (8) à l'opposé de l'embout buccal (4) ;
- un dispositif de mesure (14) comprenant une navette (16) ayant un renfoncement de dosage (15) conçu dans une face de la navette (16), dans lequel la navette (16) est mobile entre une position de remplissage, dans laquelle le renfoncement de dosage (15) se trouve en alignement avec l'ouverture (17) du récipient (7) et fait face à ladite ouverture (17) afin d'être rempli d'une dose du médicament en poudre, et une position d'inhalation, dans laquelle le renfoncement de dosage (15) se trouve en alignement avec la chambre à vortex (26) et le canal d'inhalation (8), pour permettre une inhalation de la dose du médicament en poudre contenu dans le renfoncement de dosage (15) à travers l'embout buccal (4) ;
**caractérisé en ce que**, dans une vue de dessus, l'ouverture (17) du récipient (7) est allongée le long d'un axe majeur (Y-Y) respectif et le renfoncement de dosage (15) est allongé le long d'un axe principal (X-X) respectif ; dans lequel, lorsque la navette (16) se trouve dans la position de remplissage, un bord (27) de l'ouverture (17) du récipient (7) enferme le renfoncement de dosage (15).

2. Inhalateur selon la revendication 1, dans lequel une direction de mouvement (F) de la navette (16) entre la position de remplissage et la position d'inhalation et ledit axe majeur (Y-Y) délimite entre elles un angle de 90° et dans lequel ledit axe principal (X-X) et l'axe majeur (Y-Y) délimitent entre elles un premier angle (β) autre que 0°.

3. Inhalateur selon la revendication 2, dans lequel ledit premier angle (β) est compris entre 10° et 30°.

4. Inhalateur selon l'une quelconque des revendications 1 à 3, dans lequel le désagglomérateur (25) présente deux orifices d'entrée d'air (34) s'ouvrant dans la chambre à vortex (26), les deux dits orifices d'entrée d'air (34) étant placés sur des côtés opposés de la chambre à vortex (26) et le long de directions d'écoulement d'entrée tangentielles ou sensiblement tangentielles pour former un vortex d'air dans ladite chambre à vortex (26) ; dans lequel, dans la position d'inhalation, le renfoncement de dosage (15) fait face à la chambre à vortex (26) et est entièrement enfermé dans la chambre à vortex (26) ; dans lequel le renfoncement de dosage (15) présente des extrémités opposées localisées le long de l'axe principal (X-X) ; dans lequel, dans la position d'inhalation, chacune des extrémités opposées du renfoncement de dosage (15) est proche de l'un des orifices d'entrée d'air (34).

5. Inhalateur selon la revendication 4, dans lequel une ligne dans le diamètre (Z-Z) raccordant les deux orifices d'entrée d'air (34) et l'axe principal (X-X) délimite entre eux un second angle (γ) autre que 0°.

6. Inhalateur selon la revendication 4, dans lequel le désagglomérateur (25) comprend deux parois incurvées (31) ayant des concavités se faisant face l'une l'autre, les deux parois incurvées (31) étant décalées l'une par rapport à l'autre et délimitant la chambre à vortex (26) et les deux orifices d'entrée d'air (34) tangentiels ; dans lequel une ligne dans le diamètre (Z-Z) passant à travers une extrémité libre (33) de chacune des parois incurvées (31) et de l'axe principal (X-X) délimite entre eux un second angle (γ) autre que 0°.

7. Inhalateur selon la revendication 5 ou 6, dans lequel la ligne dans le diamètre (Z-Z) est parallèle à l'axe majeur (Y-Y) et le premier angle (β) est égal au second angle (γ).

8. Inhalateur selon l'une quelconque des revendications 4 à 7, dans lequel chacune des extrémités opposées du renfoncement de dosage (15) est localisée en aval de l'orifice d'entrée d'air (34) respectif par rapport à un flux entrant d'air qui entre à travers ledit orifice d'entrée d'air (34).

9. Inhalateur selon l'une quelconque des revendications 1 à 8, dans lequel le bord (27) de l'ouverture (17) du récipient (7) est sensiblement elliptique ; ou dans lequel le bord (27) de l'ouverture (17) du récipient (7) présente deux côtés arqués majeurs et deux côtés droits mineurs.

10. Inhalateur selon l'une quelconque des revendications 1 à 9, dans lequel un périmètre du renfoncement de dosage (15) comprend deux lignes droites parallèles raccordées par deux arcs et les deux lignes droites parallèles sont parallèles à l'axe principal (X-X) ; ou dans lequel le périmètre du renfoncement de dosage (15) est ovale ou est une ellipse et l'axe principal (X-X) est un axe majeur de l'ellipse.

11. Inhalateur selon l'une quelconque des revendications 1 à 10, dans lequel le renfoncement de dosage (15) présente une capacité pour le médicament en poudre supérieure à 10 mg, éventuellement supérieure à 15 mg, éventuellement de 20 mg ; dans lequel le renfoncement de dosage (15) présente un volume supérieur à 20 mm³ éventuellement de 20 mm³ à 40 mm³ ou éventuellement de 25 mm³ à 35 mm³.

12. Inhalateur selon l'une quelconque des revendications 1 à 11, dans lequel le renfoncement de dosage (15) présente une longueur (L) mesurée le long de l'axe principal (X-X) et une largeur (W) mesurée perpendiculaire à l'axe principal (X-X) ; dans lequel un rapport L/W est supérieur à 1, éventuellement compris entre 1,4 et 1,8.

13. Inhalateur selon l'une quelconque des revendications 1 à 12, comprenant en outre un élément protecteur (18) prévu entre la navette (16) et la chambre à vortex (26) ; lorsque la navette (16) se trouve dans la position d'inhalation, l'élément protecteur (18) est mobile de manière à pouvoir coulisser sur ou au-dessus de la navette (16) entre une position fermée et une position ouverte ; dans lequel dans la position fermée l'élément protecteur (18) recouvre entièrement le renfoncement de dosage (15) empêchant une communication entre ledit renfoncement de dosage (15) et la chambre à vortex (26) ; dans lequel dans la position ouverte l'élément protecteur (18) laisse le renfoncement de dosage (15) exposé à la chambre à vortex (26).

14. Inhalateur selon l'une quelconque des revendications 1 à 13, comprenant le médicament en poudre, dans lequel ledit médicament en poudre est une composition pharmaceutique comprenant un ou plusieurs inhibiteurs de la phosphodiestérase-4 (PDE-4) sélectionnés dans le groupe constitué du tanimilaste, cilomilaste, roflumilaste, tétomilaste, oglemilaste, aprémilaste, piclamilaste et d'un sel de ceux-ci.

15. Inhalateur selon la revendication 14, dans lequel la composition pharmaceutique comprend du tanimilaste.
